# EUROPÄISCHE PATENTANMELDUNG

(11) **EP 1 555 063 A1**
(43) Veröffentlichungstag der Anmeldung: **20.07.2005**
(21) Anmeldenummer: 04024635.7
(22) Anmeldetag: 15.10.2004
(51) Int. Cl.: B01J 31/06, B01J 31/16, C07D 301/19

(54) **Katalysator für Oxidationsreaktionen und Verfahren zu dessen Herstellung**

(30) Priorität: 17.01.2004 DE 102004002497
(71) Anmelder: Forschungszentrum Karlsruhe GmbH, 76133 Karlsruhe (DE)
(72) Erfinder: Döring, Manfred, Prof. Dr., 76744 Wörth-Büchelberg (DE); Arnold, Ulrich, Dr., 76646 Bruchsal (DE)

(57) **Zusammenfassung**

Erfindungsgemäß wird ein Katalysator für Oxidationsreaktionen bestehend aus einer katalytisch wirksamen Metallverbindung, die in einem ausgehärteten duromeren Harz immobilisiert ist, vorgeschlagen. Der Katalysator wird durch ein Verfahren hergestellt, bei dem eine Verbindung eines katalytisch wirksamen Metalls eingesetzt, die Verbindung mit einem duromeren Harz vermischt und das duromere Harz mit einem Härter zu einem Duromeren ausgehärtet wird.

## Beschreibung

Die Erfindung betrifft einen Katalysator für Oxidationsreaktionen gemäß Anspruch 1 und ein Verfahren zu dessen Herstellung gemäß Anspruch 6.

Heterogen katalysierte Oxidationsreaktionen an organischen Substraten, wie z. B. Epoxidierungen und Dihyroxylierungen von Alkenen, Dehydrierungen, Oxidationen von Alkoholen, Aldehyden und Kohlenwasserstoffen, sind im allgemeinen durch niedrige Selektivitäten gekennzeichnet. Probleme bereitet insbesondere die Vergiftung des Katalysators bei unzureichender Reinheit der Reaktanden oder bei unerwünschten Nebenreaktionen.

Durch die Verwendung oxidationsaktiver Zeolithe und Alumophosphate als Matrix für katalytisch aktive Metallverbindungen konnten zwar Verbesserungen hinsichtlich der Katalysatorselektivität erreicht werden. Allerdings sind diese Katalysatoren nicht besonders stabil. Problematisch ist insbesondere, dass die katalytisch aktive Metallverbindung nach kürzerer oder längerer Zeit aus der Matrix ausgelaugt wird. Bei Zeolithen muss zudem bedacht werden, dass nur solche Reaktionen katalysiert werden können, deren Reaktanden in den Poren und Zwischenräumen des Zeoliths Platz finden. Deshalb werden auch andere Matrices hinsichtlich ihrer Eignung für Oxidationskatalysatoren überprüft.

In *Journal of Catalysis*, **183**, 251-266 (1999) beschreiben S. Leinonen et al zwei Katalysatoren für eine heterogene Epoxidierung von Alkenen. Die Katalysatoren bestehen aus einer Verbindung eines katalytisch aktiven Metalls [MoO₂(acac)₂] und einem Polymer, das im ersten Fall aus einem Polystyrol- und im zweiten Fall aus einem Polybenzimidazol-Harz besteht.

Beide Polymere sind Thermoplaste, die in aufwändigen Verfahren durch nachträgliche Modifikation bereits vorliegender Polymere dargestellt werden. Dabei werden in einem ersten Schritt koordinationschemisch aktive Gruppen in das Polymer eingebracht, an die in einem zweiten Schritt die katalytisch aktive Metallverbindung chemisch gebunden wird. Die Katalysatoren zeigen eine geringe Anwendungsbreite und es werden keine Aussagen über ihre Stabilitäten gemacht.

Der Erfindung liegt die Aufgabe zugrunde, eine weitere Matrix für katalytisch aktive Metallverbindungen vorzuschlagen. Diese Matrix soll so beschaffen sein, dass sie unter Reaktionsbedingungen stabil ist und eine ausreichend feste Wechselwirkung zur katalytisch aktiven Metallverbindung ausbildet, um ein Auslaugen der Metallverbindung zu vermeiden. Außerdem soll ein einfacheres Herstellungsverfahren für einen Katalysator für Oxidationsreaktionen vorgeschlagen werden.

Die Aufgabe wird durch den in Anspruch 1 beschriebenen Katalysator und durch das in Anspruch 6 genannte Verfahren gelöst. Die weiteren Ansprüche geben bevorzugte Ausgestaltungen des Katalysators und des Verfahrens an.

Erfindungsgemäß werden Katalysatoren vorgeschlagen, die aus einer katalytisch wirksamen Metallverbindung und einer Matrix aus einem ausgehärteten duromeren Harz bestehen. Duromere Harze können Epoxidharze, Cyanatester, Phenolharze oder Polyesterharze sein. Bevorzugt werden die beiden erstgenannten, doch sind auch Phenol- und Polyesterharze als Matrix geeignet. Die Härtung duromerer Harze erfolgt mit Hilfe eines Härters. Weitere Komponenten wie z. B. Initiatoren können die Härtung beschleunigen. Besonders bevorzugt werden solche Katalysatoren, bei denen das duromere Harz mit der katalytisch aktiven Metallverbindung oder ihrer Vorläuferverbindung (Precursor) ausgehärtet wird, so dass kein separater Härter zugegeben werden muss.

Als katalytisch wirksame Metallverbindung eignen sich praktisch alle redoxaktiven anorganischen oder organischen Verbindungen, wie z. B. organische oder anorganische Metallkomplexe, Salze, Oxide oder metallorganische Verbindungen. Wie an sich bekannt ist, sind insbesondere die Übergangsmetalle katalytisch aktiv. Soll die katalytisch wirksame Metallverbindung zugleich als Härter für das duromere Harz fungieren, eignen sich insbesondere Alkoholate, aber auch Amide, Carboxylate, Halogenide und Metallkomplexe mit 1,3-Diketonat-, Amin-, Acrylat-, Imidazol-, Imidazolium-, Phthalocyanin- und Imin-Liganden.

Ein wesentlicher Vorteil des Verfahrens zur Herstellung des Oxidationskatalysators ist seine Einfachheit. Die Metallverbindung kann unmittelbar oder in einem organischen Lösungsmittel gelöst mit dem Harz vermischt werden, wonach das Harz gehärtet wird. In der Regel wird das gehärtete Harz vor seiner Verwendung noch granuliert. Wie oben erwähnt, wird das Verfahren vorzugsweise so geführt, dass die katalytisch aktive Metallverbindung oder ihr chemischer Vorläufer (Precursor) zugleich als Härter dient. In diesem Fall spart man eine Komponente ein.

Der Katalysator weist die folgenden Vorteile auf:
- Er ist einfach und preiswert herstellbar.
- Er lässt sich in einer Vielzahl unterschiedlichen Reaktionen einsetzen.
- Die Beständigkeit gegenüber einem Auslaugen der katalytisch aktiven Metallverbindungen ist wesentlich höher als beispielsweise bei Zeolith-Matrices oder bei Thermoplasten mit koordinationschemisch aktiven Gruppen.
- Das Harz lässt sich sehr leicht vom Reaktionsgemisch abtrennen.
- Die immobilisierten katalytisch aktiven Metallverbindungen weisen in der Harzmatrix eine hohe Aktivität und Selektivität in einer Vielzahl unterschiedlicher Reaktionen auf.

Die Erfindung wird im Folgenden anhand von Ausführungsbeispielen näher erläutert.

### Beispiel 1:

### Darstellung molybdänhaltiger Epoxidharze auf Basis von 4,4'-Methylenbis(N,N-diglycidylanilin) (TGMDA) und Molybdänylacetylacetonat MoO₂(acac)₂

### Darstellung von Katalysator 1 (Polymerisation ohne Lösungsmittel)

Man suspendiert 0.457 g fein pulverisiertes MoO₂(acac)₂ in 8.699 g TGMDA und härtet die Mischung 8 h bei 180 °C in einem Ofen. Die Aluminiumform wird entfernt und das Harzplättchen (Schichtdicke ca. 2 mm) wird zunächst grob zerkleinert. Danach wird das Material mit einer Analysenmühle zermahlen. Das so erhaltene grobkörnige Pulver wird in einem Ofen 8 h bei 200 °C nachgehärtet.

### Beispiel 2:

### Darstellung von Katalysator 2 (Polymerisation mit Lösungsmittel)

Man löst 0.110 g MoO₂(acac)₂ in 2.5 ml DMF und vermischt diese Lösung mit 2.083 g TGMDA. Das Reaktionsgemisch wird in ein Aluminiumschälchen gegossen und auf einer Heizplatte bei 150 °C ausgehärtet. Nach 8 h wird ein Harzplättchen (Schichtdicke ca. 2 mm) erhalten, das 8 h auf einer Heizplatte bei 200 °C nachgehärtet wird. Nach dem Entformen wird das Plättchen zunächst grob zerkleinert und anschließend mit einer Analysenmühle gemahlen bis ein feinkörniges Granulat erhalten wird.

### Beispiel 3:

### Darstellung vanadiumhaltiger Epoxidharze auf Basis von TGMDA und Vanadylacetylacetonat VO(acac)₂

### Darstellung von Katalysator 3 (Polymerisation ohne Lösungsmittel)

Man suspendiert 0.420 g fein pulverisiertes VO(acac)₂ in 5 g TGMDA und härtet das Harz in einem Ofen 8 h bei 180°C. Nach Entfernen der Aluminiumform wird das Harzplättchen (Schichtdicke ca. 2 mm) zerschnitten und anschließend in einer Analysenmühle gemahlen. Das so erhaltene grobkörnige Pulver wird in einem Ofen 8 h bei 180 °C nachgehärtet.

### Beispiel 4:

### Darstellung von Katalysator 4 (Polymerisation mit Lösungsmittel)

Eine Lösung von 0.246 g VO(acac)₂ in 2 ml DMF wird zu 2.909 g TGMDA gegeben und dieses Gemisch wird auf einer Heizplatte 4 h bei 150 °C gehärtet. Die Aluminiumform wird entfernt und das Harzplättchen (Schichtdicke ca. 2 mm) wird nach grober Zerkleinerung in einer Analysenmühle gemahlen. Das feinkörnige Granulat wird in einem Ofen 8 h bei 230 °C nachgehärtet.

### Beispiel 5:

### Darstellung eines molybdänhaltigen Epoxidharzes auf Basis von TGMDA und Mo(OEt)₅

### Darstellung von Katalysator 5 (Polymerisation ohne Lösungsmittel)

Man löst 0.483 g Mo(OEt)₅ in 9.045 g TGMDA, gibt die Mischung in ein Aluminiumschälchen und härtet in einem Ofen. Dabei wird die Temperatur sukzessiv von 120 auf 230 °C erhöht (120 °C 1 h, 180 °C 3 h, 200 °C 2 h, 230 °C 1 h). Die Form wird entfernt, das Harzplättchen (Schichtdicke ca. 2 mm) wird zerschnitten und in einer Analysenmühle gemahlen. Das grobkörnige Pulver wird 7 h in einem Ofen bei 230 °C nachgehärtet.

### Beispiel 6:

### Epoxidierung von Cyclohexen mit tert.-Butylhydroperoxid (TBHP)

Man gibt 10.1 mmol Cyclohexen zu einer 3.2M Lösung von TBHP in Toluol (16.2 mmol TBHP) und setzt 500 mg Katalysator zu. Das Reaktionsgemisch wird auf 90 °C erwärmt und 7 h bei dieser Temperatur gehalten. Man lässt auf Raumtemperatur abkühlen und trennt den Katalysator durch Filtration ab.
Die Produktverteilung des Reaktionsgemischs wird mittels GC-MS und GC-FID bestimmt. Zur Untersuchung der Katalysatorstabilität wird das abgetrennte Harz in mehreren aufeinanderfolgenden Reaktionen eingesetzt. Metallgehalte der Reaktionslösungen werden spektroskopisch mittels ICP-AES ermittelt. Hierzu werden die flüssigen Bestandteile abdestilliert und die Rückstände werden in konz. HNO₃ aufgenommen.

| Katalytische Aktivität von Katalysator **1** bei wiederholtem Einsatz in der Epoxidierung von Cyclohexen^{a} | | | | | | | | | |
|---|---|---|---|---|---|---|---|---|---|
| Reaktion Nr. | Umsatz^{b} (%) | Selektivität^{b} (%) | | | | | Mo-Verlust^{c} (µg) | TN/ Mo total^{d} | TN/ Mo gelöst^{d} |
| | | Epoxid | Cyclohexenol | Cyclohexenon | Diol | Andere | | | |
| Blank | 33 | 7 | 10 | 7 | 76 | - | - | - | - |
| 1 | > 99 (66)^{e} | 88 | < 1 | 1 | 9 | 2 | 972 | 87 | 700 |
| 2 | > 99 (66) | 94 | < 1 | 1 | 3 | 2 | 132 | 100 | 4800 |
| 3 | > 99 (66) | 93 | < 1 | 1 | 2 | 3 | 37 | 102 | 17300 |
| 4 | 98 (65) | 88 | < 1 | 2 | 5 | 5 | 22 | 101 | 28600 |
| 5 | 94 (61) | 83 | 1 | 3 | 7 | 6 | 14 | 95 | 42200 |
| 6 | 93 (60) | 86 | 1 | 2 | 6 | 5 | 19 | 94 | 30600 |
| 7 | 81 (48) | 77 | 3 | 4 | 11 | 5 | 15 | 76 | 31000 |
| 8 | 83 (50) | 81 | 2 | 3 | 9 | 5 | 13 | 79 | 37300 |
| 9 | 97 (64) | 91 | < 1 | 2 | 3 | 3 | 22 | 101 | 28200 |
| 10 | 68 (35) | 71 | 4 | 4 | 17 | 4 | 9 | 56 | 37700 |

| | | | | | | | | | |
|---|---|---|---|---|---|---|---|---|---|
| ^{a}Reaktionsbedingungen: 10.1 mmol Cyclohexen, 16.2 mmol TBHP (3.2M Lösung in Toluol), 500 mg Katalysator (7.35 mg Mo, 7.66×10⁻⁵ mol Mo, 0.76 mol%, Molverhältnis Metall:Alken 1:131), 363 K, 7 h | | | | | | | | | |
| ^{b}Bestimmung mittels GC-FID und GC-MS mit Dodecan als externem Standard | | | | | | | | | |
| ^{c}bestimmung mittels ICP-AES aus Lösungen der Reaktionsrückstände in konzentrierter HNO₃ | | | | | | | | | |
| ^{d}TN = ∑mol(Produkte)/mol(Katalysator), alle Werte blank-korrigiert | | | | | | | | | |
| ^{e}Blank-korrigierte Werte in Klammern | | | | | | | | | |

| Katalytische Aktivität von Katalysator **2** bei wiederholtem Einsatz in der Epoxidierung von Cyclohexen^{a} | | | | | | | | | |
|---|---|---|---|---|---|---|---|---|---|
| Reaktion Nr. | Umsatz^{b} (%) | Selektivität^{b} (%) | | | | | Mo-Verlust^{c} (µg) | TN/ Mo total^{d} | TN/ Mo gelöst^{d} |
| | | Epoxid | Cyclohexenol | Cyclohexenon | Diol | Andere | | | |
| Blank | 33 | 7 | 10 | 7 | 76 | - | - | - | - |
| 1 | 67 (34)^{e} | 64 | 7 | 5 | 22 | 2 | 18 | 45 | 18300 |
| 2 | 96 (63) | 89 | 1 | 2 | 4 | 4 | 107 | 83 | 5700 |
| 3 | > 99 (66) | 93 | < 1 | 1 | 3 | 2 | 135 | 89 | 4700 |
| 4 | > 99 (66) | 93 | < 1 | 2 | 1 | 3 | 67 | 90 | 9500 |
| 5 | 99 (66) | 94 | < 1 | 2 | 2 | 2 | 38 | 91 | 16800 |
| 6 | 98 (65) | 89 | < 1 | 3 | 4 | 4 | 41 | 90 | 15400 |
| 7 | 99 (66) | 93 | 1 | 3 | 1 | 2 | 83 | 92 | 7700 |
| 8 | > 99 (66) | 93 | 1 | 2 | 1 | 3 | 76 | 93 | 8400 |
| 9 | > 99 (66) | 93 | < 1 | 2 | 3 | 2 | 60 | 94 | 10700 |
| 10 | 99 (66) | 90 | < 1 | 2 | 3 | 4 | 59 | 95 | 10800 |

| | | | | | | | | | |
|---|---|---|---|---|---|---|---|---|---|
| ^{a}Reaktionsbedingungen: 10.1 mmol Cyclohexen, 16.2 mmol TBHP (3.2M Lösung in Toluol), 500 mg Katalysator (7.35 mg Mo, 7.66×10⁻⁵ mol Mo, 0.76 mol%, Molverhältnis Metall:Alken 1:131), 363 K, 7 h | | | | | | | | | |
| ^{b}Bestimmung mittels GC-FID und GC-MS mit Dodecan als externem Standard | | | | | | | | | |
| ^{c}Bestimmung mittels ICP-AES aus Lösungen der Reaktionsrückstände in konzentrierter HNO₃ | | | | | | | | | |
| ^{d}TN = ∑mol(Produkte)/mol(Katalysator), alle Werte blankkorrigiert | | | | | | | | | |
| ^{e}Blank-korrigierte Werte in Klammern | | | | | | | | | |

| Katalytische Aktivität von Katalysator 3 bei wiederholtem Einsatz in der Epoxidierung von Cyclohexen^{a} | | | | | | | | | | |
|---|---|---|---|---|---|---|---|---|---|---|
| Reaktion Nr. | Umsatz^{b} (%) | Selektivität^{b} (%) | | | | | | v-Verlust^{c}(µg) | TN/ V total^{d} | TN/ V gelöst^{d} |
| | | Epoxid | Cyclohexenol | Cyclohexenon | Cyclohexenonoxid | Diol | Andere | | | |
| Blank | 33 | 7 | 10 | 7 | - | 76 | - | - | - | - |
| 1 | 86 (53)^{e} | 49 | 1 | 13 | 2 | 7 | 28 | 688 | 37 | 400 |
| 2 | 89 (56) | 50 | 2 | 13 | 2 | 8 | 25 | 760 | 43 | 380 |
| 3 | 88 (55) | 45 | 1 | 15 | 2 | 8 | 29 | 493 | 47 | 570 |
| 4 | 83 (50) | 44 | 1 | 16 | 2 | 7 | 30 | 340 | 47 | 760 |
| 5 | 79 (46) | 44 | 2 | 15 | 2 | 8 | 29 | 232 | 46 | 1020 |

| | | | | | | | | | | |
|---|---|---|---|---|---|---|---|---|---|---|
| ^{a}Reaktionsbedingungen: 10.1 mmol Cyclohexen, 16.2 mmol TBHP (3.2M Lösung in Toluol), 500 mg Katalysator (7.45 mg V, 1.46×10⁻⁴ mol V, 1.44 mol%, Molverhältnis Metall:Alken 1:69), 363 K, 7 h | | | | | | | | | | |
| ^{b}Bestimmung mittels GC-FID und GC-MS mit Dodecan als externem Standard | | | | | | | | | | |
| ^{c}Bestimmung mittels ICP-AES aus Lösungen der Reaktionsrückstände in konzentrierter HNO₃ | | | | | | | | | | |
| ^{d}TN = Emol(Produkte)/mol(Katalysator), alle Werte blank-korrigiert | | | | | | | | | | |
| ^{e}Blank-korrigierte Werte in Klammern | | | | | | | | | | |

| Katalytische Aktivität von Katalysator **4** bei wiederholtem Einsatz in der Epoxidierung von Cyclohexen^{a} | | | | | | | | | | |
|---|---|---|---|---|---|---|---|---|---|---|
| Reaktion Nr. | Umsatz^{b} (%) | Selektivität^{b} (%) | | | | | | V-Ver-lust^{c} (µg) | TN/ V total^{d} | TN/ V gelöst^{d} |
| | | Epoxid | Cyclohexenol | Cyclohexenon | Cyclohexenonoxid | Diol | Andere | | | |
| Blank | 33 | 7 | 10 | 7 | - | 76 | - | - | - | - |
| 1 | 66 (33)^{e} | 46 | 2 | 15 | 2 | 8 | 27 | 172 | 23 | 990 |
| 2 | 74 (41) | 46 | 1 | 15 | 2 | 7 | 29 | 258 | 29 | 820 |
| 3 | 77 (44) | 46 | 1 | 16 | 2 | 7 | 28 | 311 | 32 | 730 |
| 4 | 74 (41) | 46 | 1 | 16 | 2 | 6 | 29 | 308 | 31 | 690 |
| 5 | 81 (48) | 46 | 1 | 17 | 2 | 6 | 28 | 401 | 38 | 620 |

| | | | | | | | | | | |
|---|---|---|---|---|---|---|---|---|---|---|
| ^{a}Reaktionsbedingungen: 10.1 mmol Cyclohexen, 16.2 mmol TBHP (3.2M Lösung in Toluol), 500 mg Katalysator (7.50 mg V, 1.47×10⁻⁴ mol V, 1.46 mol%, Molverhältnis Metall:Alken 1:69), 363 K, 7 h | | | | | | | | | | |
| ^{b}Bestimmung mittels GC-FID und GC-MS mit Dodecan als externem Standard | | | | | | | | | | |
| ^{c}Bestimmung mittels ICP-AES aus Lösungen der Reaktionsrückstände in konzentrierter HNO₃ | | | | | | | | | | |
| ^{d}TN = ∑mol(Produkte)/mol(Katalysator), alle Werte blankkorrigiert | | | | | | | | | | |
| ^{e}Blank-korrigierte Werte in Klammern | | | | | | | | | | |

| Katalytische Aktivität von Katalysator 5 bei wiederholtem Einsatz in der Epoxidierung von Cyclohexen^{a} | | | | | | | |
|---|---|---|---|---|---|---|---|
| Reaktion Nr. | Umsatz^{b} (%) | Selektivität^{b} (%) | | | | | TN/ Mo total^{c} |
| | | Epoxid | Cyclohexenol | Cyclohexenon | Diol | Andere | |
| Blank | 33 | 7 | 10 | 7 | 76 | - | - |
| 1 | > 99 (66)^{d} | 98 | - | < 1 | < 1 | 1 | 84 |
| 2 | 96 (63) | 98 | - | < 1 | < 1 | 1 | 81 |
| 3 | 92 (59) | 98 | < 1 | < 1 | < 1 | 1 | 75 |
| 4 | 89 (56) | 97 | < 1 | < 1 | 1 | 1 | 72 |
| 5 | 86 (53) | 98 | < 1 | < 1 | < 1 | 1 | 68 |
| 6 | 84 (51) | 98 | < 1 | < 1 | < 1 | 1 | 65 |
| 7 | 83 (50) | 97 | < 1 | < 1 | 1 | 1 | 64 |
| 8 | 82 (49) | 98 | < 1 | < 1 | < 1 | 1 | 63 |
| 9 | 83 (50) | 97 | < 1 | < 1 | 1 | 1 | 64 |
| 10 | 82 (49) | 97 | < 1 | < 1 | 1 | 1 | 63 |

| | | | | | | | |
|---|---|---|---|---|---|---|---|
| ^{a}Reaktionsbedingungen: 10.1 mmol Cyclohexen, 16.2 mmol TBHP (3.2M Lösung in Toluol), 500 mg Katalysator (7.57 mg Mo, 7.89×10⁻⁵ mol Mo, 0.78 mol%, Molverhältnis Metall:Alken 1:128), 363 K, 7 h | | | | | | | |
| ^{b}Bestimmung mittels GC-FID und GC-MS mit Dodecan als externem Standard | | | | | | | |
| ^{c}TN = ∑mol(Produkte)/mol(Katalysator), alle Werte blank-korrigiert | | | | | | | |
| ^{d}Blank-korrigierte Werte in Klammern | | | | | | | |

## Patentansprüche

1. Katalysator für Oxidationsreaktionen bestehend aus einer katalytisch wirksamen Metallverbindung, die in einem ausgehärteten duromeren Harz immobilisiert ist.

2. Katalysator nach Anspruch 1 mit einem Epoxidharz oder einem Cyanatester als duromeres Harz.

3. Katalysator nach Anspruch 1 oder 2, bei dem das duromere Harz mit der katalytisch wirksamen Metallverbindung oder einer Vorläuferverbindung (Precursor) der Metallverbindung gehärtet worden ist.

4. Katalysator nach Anspruch 2 oder 3 mit dem Diglycidylether von Bisphenol A (DGEBA) oder einem Tetraglycidyl-Derivat von Methylendianilin (TGMDA) oder einem Novolak als Epoxidharz.

5. Katalysator nach Anspruch 2 oder 3 mit einem Bisphenol-Adicyanat oder einem Polyphenolcyanat als Cyanatester.

6. Verfahren zur Herstellung eines Katalysators für Oxidationsreaktionen, bei dem eine Verbindung eines katalytisch wirksamen Metalls eingesetzt,
die Verbindung mit einem duromeren Harz vermischt und das duromere Harz mit einem Härter zu einem Duromeren ausgehärtet wird.

7. Verfahren nach Anspruch 6, bei dem die Verbindung des katalytisch wirksamen Metalls als Härter für das duromere Harz eingesetzt wird.

8. Verfahren nach Anspruch 6 oder 7, bei dem die Verbindung des katalytisch wirksamen Metalls ein Metallalkoholat oder ein Metall-1,3-Dikentonat ist.
